# EUROPEAN PATENT SPECIFICATION

(11) **EP 4 296 213 B1**
(45) Date of publication and mention of the grant of the patent: **04.06.2025**
(21) Application number: 22717251.7
(22) Date of filing: 21.02.2022
(51) Int. Cl.: B66B 31/02

(54) **SYSTEM FOR CLEANING AND DISINFECTING HANDRAILS OF ESCALATORS AND MOVING WALKWAYS**
SYSTEM ZUR REINIGUNG UND DESINFEKTION VON HANDLÄUFEN VON FAHRTREPPEN UND FAHRSTEIGEN
SYSTÈME DE NETTOYAGE ET DE DÉSINFECTION DE MAINS COURANTES D'ESCALIERS ET DE TAPIS ROULANTS

(30) Priority: 19.02.2021 PT 2021117077
(43) Date of publication of application: 27.12.2023
(73) Proprietor: Escola Superior de Enfermagem de Coimbra, 3001-901 Coimbra (PT); Instituto Politécnico de Coimbra, 3000-271 Coimbra (PT)
(72) Inventor: SANTOS DINIS PARREIRA, Pedro Miguel, 3045-293 Ameal (PT); SOUSA SALGUEIRO OLIVEIRA, Anabela, 3030-175 Coimbra (PT); SANTOS PEREIRA MALÇA, Cândida Maria, 3000-135 Coimbra (PT); GARCIA NASCIMENTO GRAVETO, João Manuel, 3000-194 Coimbra (PT); FIGUEIREDO AGOSTINHO ABREU MENDES, Fernando José, 3080-017 Figueira Da Foz (PT); ALMEIDA OSÓRIO, Nádia Isabel, 3140-331 Pereira MMV (PT)
(74) Representative: Moniz Pereira, Manuel
(86) International application number: PCT/IB2022/051518
(87) International publication number: WO 2022/175912

(56) References cited:
- EP-A1- 3 889 093
- WO-A2-2010/027139
- CN-A- 102 134 039
- KR-A- 20110 100 040

## Description

### TECHNICAL DOMAIN

The present invention concerns an automatic system for cleaning and disinfecting all configurations of handrails of escalators and moving walkways.

### BACKGROUND

It is considered crucial to implement innovative measures to control the spread of microorganisms through surfaces such as handrails of escalators and moving walkways to protect public health. The disinfection systems available on the market do not promote the total elimination of microorganisms, since they do not perform the mechanical removal of the surface of the handrail which would promote the cleaning and subsequent elimination of organic matter/dirt present on the surface. Commercially available systems apply substances and/or emit energy sources (ultraviolet/infrared) that promote the destruction of only part of the microorganisms. These systems do not perform mechanical removal, and as such it is not possible to adequately disinfect the surface due to the inability of such energy sources and chemical agents to penetrate it, due to the presence of organic matter (glycocalyx). Further, it should be noted that some available systems also do not allow for easy integration into every type of escalator or moving walkway, and there are no standard systems available that can be applied to most escalators and moving walkways.

Patents WO2012149475 and US20130240756, which do not enable the possibility of being applied to every type of escalator or moving walkway, i.e., they are not easily adjustable and do not present a standard model applicable to most escalators and moving walkways, are worth mentioning. Additionally, they do not provide cumulatively nor sequentially the functions of cleaning and disinfection through initial wet mechanical removal of the handrail surface, spraying with disinfectant, ultraviolet light action, and drying through heat source. Also WO 2010/027139 A2 and CN 102134039 A are disclosing relevant handrail cleaning devices.

### GENERAL DESCRIPTION

Inanimate objects play an important role in the transmission of microorganisms, mostly from humans, and through the hands or from the microbiota of the skin. Many of these microorganisms have a high potential for adhesion to surfaces, and their attachment depends only on the existence of an exopolysaccharide matrix rich in nucleic acids, proteins, minerals, and nutrients, which will act as "glue", called glycocalyx. Subsequently, microbial multiplication occurs on the surface, forming a biofilm, which leads to increased metabolic efficiency of the microbial community, allows gene exchange resulting in more virulent microorganisms, protects microorganisms against antimicrobial agents, and enables the formation of aggregates that easily spread either to other locations, or to other individuals.

Transmission can easily occur by contact, with hand contamination and subsequent contact with the mouth, eyes, nose, and skin being the most common route, or through droplets, in the case of microorganisms larger than 5 µm, expelled by speaking, sneezing, or coughing that are deposited on surfaces, e.g., on handrails. It is known that many of these microorganisms can remain on surfaces for hours, days, or weeks, and have pathogenic potential when transmitted to children, the elderly, and/or immunosuppressed individuals, causing gastrointestinal, respiratory, and other infectious diseases, where we can easily fit the current COVID-19 pandemic.

The present invention solves the above problems since with its implementation and use the cleaning section is achieved through wet mechanical removal, promoting the removal by friction of the extracellular matrix, avoiding microbial adhesion and potential formed microbial biofilm. The contact of an appropriately textured fabric on the surface of the handrail, soaked in disinfectant fluid, maintains and ensures its integrity, promoting disinfection. Incorporating antimicrobial nanoparticles into the cleaning and disinfection solution or incorporating them into the elastomer, which embodies the handrail, will contribute to disinfection by releasing the nanoparticles incorporated in the material; drying of the handrail surface occurs after it passes through the ultraviolet (UV) light and heat source for drying. In this way, in addition to not causing discomfort to the user, this process enables, simultaneously, the evaporation of the disinfectant liquid using natural convection (if the environment itself is sufficient to cause the evaporation of the fluid), or by forced convection (e.g., electric dryer) or by means of equipment capable of generating heat through radiation (e.g., electric resistances), while respecting the time necessary for the disinfectant to perform its germicidal action.

Thus, the present invention presents the following advantages vis-à-vis the state of the art:
- removal of organic matter aimed at reducing the matrix that promotes microbial adhesion, by wet mechanical cleaning action of cleaning and disinfection substance, of most of the bacterial load present on the surface of any configuration of handrails of escalators and moving walkways, which will consequently decrease microbial concentration;
- chemical disinfection process to eliminate any remaining microorganisms through direct-contact chemical disinfection of a disinfectant, thus reducing the transmission of the contamination chain between individuals, a potential risk of opportunistic infection in the most vulnerable citizens, promoting greater safety for users of handrails attached to moving walkways or escalators in public spaces, with an increase in Public Health;
- drying system through a heat source;
- system that allows the collection of samples for subsequent monitoring of the microbial load and evaluation of the effectiveness of the disinfection process and corresponding registration;
- removal of glycocalyx and/or microbial biofilm and disinfection;
- autonomous operation, which reduces the need for human intervention or interruption of operation;
- easy adaptation and application to any type and configuration of escalator or moving walkway, enabling the disinfection of the surface of any configuration of handrails of escalators and moving walkways and ensuring that its maintenance is low-cost and easy to perform;
- adjustment to any type of escalator or moving walkway, i.e., any angle of inclination that it might present necessarily makes a difference in the geometry of the handrail;
- replacement system in kit form;
- incorporation of automatic reading and sampling mechanisms - for evaluation of disinfection effectiveness for maintenance reporting, such as a remote-control panel for automatic operation of the disinfection system;
- possibility of using the sides of the system enclosure for advertising.

It is worth mentioning that, from an ecological point of view, an attempt is being made to solve a Public Health problem by using environmentally friendly materials, since recyclable materials and/or industry surpluses (waste) are used in the production of the present invention, as is the case, for example, with the use of thermoplastics such as polyethylene, which can come from recycling bottles, and others reinforced with, e.g., wood fibres, known as sawdust, or particles coming from glass recycling.

The present invention concerns a system for cleaning and disinfecting handrails of escalators and moving walkways that automatically enables the cleaning and disinfection of the surface of any configuration of handrails of escalators and moving walkways, by removing the larger particles from the surface of the handrail, by the action of a mechanism wherein two shafts work together to unwind and wind fabric with specific characteristics to eliminate the bacterial load, ensuring contact with the surface of the handrail, and thus carrying out its mechanical removal by friction, through the contact of appropriate fabric, soaked in a cleaning/disinfectant solution. After this disinfection, it follows the deposition of a disinfectant product of small droplets through direct contact of the surface of the handrail with a fabric with characteristics suitable for the purpose, enabling disinfection through contact of the surface of the handrail with fabric soaked in a disinfectant solution through a mechanism in which two shafts work together to unwind and wind fabric with specific characteristics to eliminate the bacterial load, ensured through contact of the surface of the handrail with the fabric that has appropriate characteristics and is soaked in disinfectant solution. Subsequently, a natural drying of the surface by convection or heat source takes place, preventing users from feeling the discomfort of contact with a damp surface, with the disinfection being assisted by the anti-germicidal action of ultraviolet light (UV), i.e., the surface of the handrail is simultaneously submitted to the action of UV lights.

### BRIEF DESCRIPTION OF THE FIGURES

In a preferred embodiment:

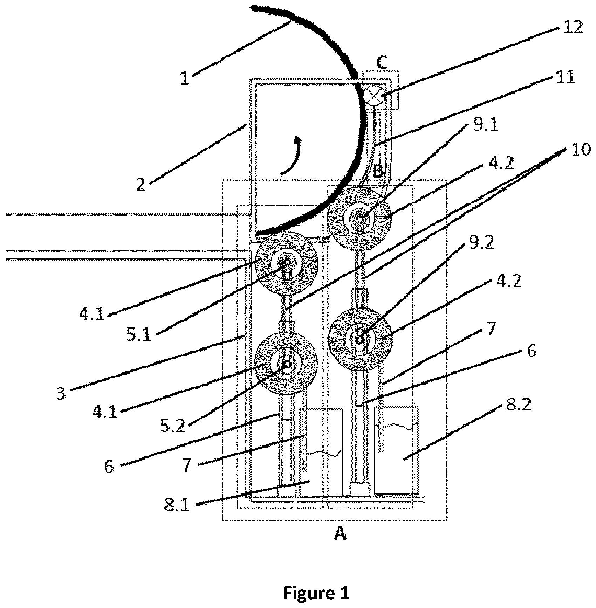

**Figure** 1 - Representation of an embodiment of the present invention showing the cleaning module (A), which is comprised of two blocks, one that promotes the removal, comprising a pair of shafts of the first block (5), which promote the rotation of two reels of a fabric of the first block (4.1), through drive belts (10) that connect the shafts of the first block (5) and that are driven by a at least synchronized electric motor (6). A tank of the first block (8.1), to which a hose (7) with an attached injector that sprays droplets of a disinfectant liquid is connected; a second block, which carries out the disinfection, comprising a pair of shafts of the second block (9), which promote the rotation of two reels of a fabric of the second block (4.2) through drive belts (10) that connect the shafts of the second block (9) and that are driven by the at least one synchronized electric motor (6). It features a tank of the second block (8.2), to which a hose (7) with an attached injector that sprays droplets of a disinfectant liquid is connected. The module for subjecting the handrail surface to ultraviolet light (B) action comprises at least one ultraviolet (UV) light source (11) and the drying module (C), which promotes drying by convection through at least one dryer or through at least one heat source (12).

The elements and components of the device of the present invention are marked in the figures:
A. cleaning module
   1. handrail
   2. upper side
   3. lower side
   4. fabrics
      4.1. fabric of the first block
      4.2. fabric of the second block
   5. shafts of the first block
      5.1. upper shaft of the first block
      5.2. lower shaft of the first block
   6. electric motor
   7. hose
   8. tanks
      8.1. tank of the first block
      8.2. tank of the second block
   9. shafts of the second block
      9.1. upper shaft of the second block
      9.2. lower shaft of the second block
   10. drive belts
B. module for subjecting the handrail surface to ultraviolet light
   11. light source
C. drying module
   12. heat source

### DETAILED DESCRIPTION

The present invention concerns a system for cleaning and disinfecting handrails of escalators and moving walkways, which comprises three modules properly compartmentalized so that there is no functional interference between them:
A. cleaning module, in which mechanical rubbing and disinfection is performed;
B. module for subjecting the surface of the handrail to ultraviolet light;
C. drying module, which seeks to dry the surface of the handrail, i.e., to promote the drying of the disinfectant liquid by convection (which might be natural, if the environment itself is sufficient to cause the evaporation of the fluid, therefore not causing any discomfort to the user as they will not find the surface damp);
the coupling of the three modules that make up the system that is the object of the invention is carried out directly on the handrail to be cleaned and coupled to the structure of the escalator or moving walkway, of which the handrail is an integral part, through its horizontal and vertical adjustment to ensure perfect coupling with the structure.

The cleaning module (A) comprises two blocks that are similar in their constitution, but with different functions:
- a first block promoting the removal, comprising a pair of shafts of the first block (5), a lower shaft of the first block (5.2), and an upper shaft of the first block (5.1), which promote the rotation of two reels of one of the fabrics (4), a fabric of the first block (4.1), one each, through drive belts (10) that connect the shafts of the first block (5) and that are driven by at least one electric motor (6) synchronized with the rotation of the escalator or moving walkway, providing the winding/unwinding of the fabric of the first block (4.1). The shafts of the first block (5) are positioned vertically such that the fabric of the first block (4.1), as it moves, contacts the surface of the handrail (1), removing larger debris from the surface of the handrail (1). Removal is accomplished through friction, i.e., the friction generated, or direct contact, between the surface of the handrail (1) and the fabric of the first block (4.1), this fabric of the first block (4.1) having a texture and characteristics suitable to enable the removal of organic matter and/or dirt, microbial adhesion matrix, and any biofilm formed. It also presents one of the tanks (8), a tank of the first block (8.1), to which a hose (7) is connected, with an attached injector that sprays droplets of a disinfectant liquid, which is in the tank of the first block (8.1), onto the fabric of the first block (4.1), impregnating the fabric of the first block (4.1), which will be in contact with the surface of the handrail (1), with the disinfectant liquid and by action of the friction generated, promoting the removal of undesirable matter from the surface of the handrail (1);
- a second block, which carries out the disinfection, comprising a pair of shafts of the second block (9), an upper shaft of the second block (9.1) and a lower shaft of the second block (9.2), which promote the rotation of two reels, one each, of one of the fabrics (4), a fabric of the second block (4.2) through drive belts (10) that connect the shafts of the second block (9) and that are driven by at least one electric motor (6) synchronized with the rotation of the escalator or moving walkway, providing the winding/unwinding of the fabric of the second block (4.2). These shafts of the second block (9) are positioned vertically such that the fabric of the second block (4.2), as it moves, contacts the surface of the handrail (1), disinfecting the surface of the handrail (1). It further has a tank of the second block (8.2), to which a hose (7) is connected, with an attached injector that sprays droplets of a disinfectant liquid, which is in one of the tanks (8), in a tank of the second block (8.2), onto the fabric of the second block (4.2), impregnating the fabric of the second block (4.2), which will be in contact with the surface of the handrail (1), with disinfectant liquid, thus promoting disinfection.

In a preferred embodiment, the shafts of the first block (5) and the shafts of the second block (9) are removable, i.e., it is possible to remove the shafts to replace the fabric (4) reels without uncoupling the drive belt (10) from the at least one electric motor (6), which transmits power.

In a preferred embodiment of the invention, the tanks (8) are placed at the level of the start of the escalator or moving walkway, or directly on the floor of the escalator or moving walkway, or on a base of a structure supporting the system which is the object of the present invention. In another alternative embodiment, the tanks (8) can be placed on the support structure of the moving walkway or escalator itself, optimizing the footprint of the cleaning system that is the object of the present invention.

The cleaning and disinfection system that is the object of the present invention is supported at the level of the moving walkway or escalator to be used and, as such, its weight is supported directly by the ground or surface where the moving walkway or escalator is installed, instead of being supported directly by the structure of the handrail (1), having the advantage of being more discreet, not being an extra weight for the structure of the escalator or moving walkway, and having the possibility of being surrounded by a structure comprising at least one upper side (2) and at least one lower side (3) that, in addition to serving as protection for users and the system itself, may also be used as a means of advertising.

It should be noted that the fact that each block of the cleaning and disinfection module (A) comprises two reels, i.e., the fabrics (4) run in a circuit that passes between two reels and the surface of the handrail (1), thus ensuring that the surface of the fabrics (4), which either promotes friction on the surface of the handrail (1) or disinfects it, is always new or clean and as such, free of contamination. In short, there is always an amount of each clean fabric (4) in contact with the handrail (1), thus ensuring scraping and disinfection. Thus, the fabrics (4) pass in the reels of the upper shaft of the first block (5.1) and the upper shaft of the second block (9.1), respectively, when new or clean and the fabrics (4) are housed in the reels of the lower shaft of the first block (5.2) and the lower shaft of the second block (9.2), respectively, when used or contaminated. Both fabrics (4) have special characteristics in their structure and properties: they can be Taffeta, Serge, or Satin with maximum friction strength, capacity for impregnation, and are highly hydrophilic with the capability to sense and incorporate, namely, among several possibilities, the integration of antimicrobial nanoparticles. In order to fulfil the purpose of scraping, the fabric of the first block (4.1) has different characteristics, such as roughness, from the fabric of the second block (4.2), which disinfects the surface. Additionally, the chemicals impregnated in each of the fabrics (4) can have different characteristics among them.

It is also important to mention that it is essential that the scraping block is the first block to act, followed by the disinfection block, so the relative location of these two blocks must ensure this sequence of steps. Thus, it is necessary that the placement of the scraping block of module (A) be located so that it contacts the surface of the moving walkway or escalator before the disinfection module does.

Since a roller system is used that holds new/clean fabrics and used/contaminated fabrics, only one side of the fabrics (4) contacts the handrail (1) on the first pass. In this way, the second side of the fabrics (4) will be reused, if necessary, constituting an added value for the present invention, because twice the usage will be possible with the same amount of fabric, prolonging the operation of the system, with a resulting smaller need for human intervention. Even more specifically, it is detailed that the present invention comprises two tanks (8) or reservoirs, each tank (8) with two shafts of the first block (5) and two shafts of the second block (9), respectively, which hold two reels that are connected (not shown in the figure); this connection is made through the fabrics (4), respectively. When the escalator is in motion, the two reels that are in direct contact with the escalator, i.e., those supported by the upper shaft of the first block (5.1) and by the upper shaft of the second block (9.1), unwind the fabrics (4), which wind onto the other windings that are supported by the lower shaft of the first block (5.2) and the lower shaft of the second block (9.2), with the fabrics (4) thus being in permanent strain. To keep the fabrics (4) in direct contact with the handrail (1), there is a tension system to always keep the fabrics (4) in contact with the handrail (1), as the reel (which is in contact with the handrail (1) unwinds and loses volume/diameter.

For the removal phase (scraping/friction) to be complete, the type of fabric and its size are determining factors. Scraping is the mechanism that is the first frontier for any debris present on the handrail (1) as well as a first step in removing organic debris and/or microorganisms. Thus, the fabric of the first block (4.1), for this scraping step is non-abrasive to the surface of the handrail (1) and its dimensions suit the size of the reels passing through the shafts of the first block (5) of fabric of the first block (4.1). The system will operate according to a pre-set schedule, i.e., the new surface does not necessarily have to be permanently in operation and in contact with the handrail (1) but can be programmed according to a set operating schedule. In this way, it will be possible to optimize the gradual unwinding of the fabric (4) by optimizing the exact dimensions of the reels. Finally, the optimization of the support of the fabric (4) on the handrail (1) is crucial, and it is an asset to design the support with the shape of the handrail (1) section to scrape the entire surface.

It should be noted that the installation conditions of the present invention only require a first and single adjustment throughout its functional life, as the configurations of the escalator and moving walkways are constant. To achieve this effect, a regulation by means of screws is integrated, sliding on several rails that together provide horizontal and vertical movement of each phase of the system.

The cleaning module (A) does not ensure that pathogenic microorganisms are completely removed from the surface of the handrail (1) and/or completely eliminated. It only reduces the matrix that can support microbial adhesion and its subsequent installation in bacterial biofilm. To solve this problem, the present invention comprises additional disinfection zones, such as the ultraviolet (UV) action responsible for eliminating the remaining microorganisms.

The module for subjecting the handrail surface to ultraviolet light (B) comprises at least one ultraviolet (UV) light source (11) for subjecting the handrail (1) surface to it. This light source (11) is also powered by the at least one electric motor (6) that drives the fabric (4) reels. Module for subjecting the handrail surface to ultraviolet light (B) must be located so that its action takes place after the disinfection step performed by module (A) and before the action of the drying module (C).

The drying module (C) has the purpose of drying the surface of the handrail (1), i.e., to promote the drying of the disinfectant liquid. This drying module (C), in a preferred embodiment, promotes drying by convection through at least one dryer or through at least one heat source (12), e.g., the use of at least one electrical resistance, ensuring that no discomfort is caused to the user as they will not find the surface damp. Regardless of the heat production source selected, it will be driven by the same at least one electric motor (6) that triggers the drive belts (10) of the fabric (4) reels.

Drying module (C) must be located so that its action takes place after the action step of module for subjecting the handrail surface to ultraviolet light (B).

It is understood that the present invention can be adjusted horizontally by means of the distance between the two constituent blocks of the cleaning and disinfection module (A). This distance may be the one that best suits the escalator or moving walkway to be disinfected. Vertical adjustment is also possible, and this can be independent between each of the blocks, or be in tandem, i.e., both blocks always have the same displacement, thus guaranteeing that the space between them is always equal.

These adjustments are made using methodologies known in the state of the art, e.g., screws in holes that, once tightened with nuts, prevent any movement, stabilizing the device. These screws are present in the various adjustment rails.

The present device further comprises angle poles and pre-drilled bars, which constitute the structural part, made of metal or any other type of material with the desirable mechanical resistance. All these elements are joined and fixed by bolted connections consisting of screws, nuts, and washers.

It is thus possible to perform the functions of adjustment to any type of handrail/moving walkway configuration, to continuously provide a new surface when scraping it, to use the same fabric (4) reel for two different uses, and where the corresponding replacement is achieved without affecting the rest of the system's operation. Its autonomous operation is also ensured without the need for human intervention, for long periods of time, including maintenance interventions for the continued operation of the device. Finally, the space for electrical applications is well defined in the structure.

The present invention can be constructed from construction steel, plastic, or using composite materials produced from recycling undifferentiated plastic materials reinforced with plant fibres.

As will be evident to a person skilled in the art, the present invention should not be limited to the embodiments described herein, and various modifications are possible that remain within the scope of the present invention defined by the claims.

## Claims

1. Cleaning and disinfection system for handrails of escalators and moving walkways wherein the system comprising a cleaning module (A), a module for subjecting the surface of a handrail to ultraviolet light (B), and a drying module (C), wherein:
a. the cleaning module (A) comprises two blocks:
i. a first block, comprising a pair of shafts of the first block (5), which comprise an upper shaft of the first block (5.1) and a lower shaft of the first block (5.2), a second block comprising a pair of shafts of the second block (9), which comprise an upper shaft of the second block (9.1) and a lower shaft of the second block (9.2), which promote the rotation of two fabric (4) reels, one each block, by means of drive belts (10) that connect the shafts of the first block (5) and the shafts of the second block (9) and that are driven by at least one electric motor (6) synchronized with the rotation of the escalator or moving walkway, providing the winding/unwinding of the fabrics (4);
ii. tanks (8) connected to hoses (7) that have coupled injectors that spray the fabrics (4);
iii. the shafts of the first block (5) and the shafts of the second block (9) are positioned vertically in such a way that the fabrics (4), when moving, contact the surface of a handrail (1), removing larger debris from the surface of the handrail (1) and then disinfecting the surface of the handrail (1) respectively;
b. module for subjecting the handrail surface to ultraviolet light (B), which comprises at least one ultraviolet (UV) light source (11) and located such that its action occurs after the disinfection step performed by the cleaning module (A) and before the action of the drying module (C), said drying module (C) comprising at least one dryer or at least one heat source (12).

2. System according to the preceding claim wherein the shafts of the first block (5) and the shafts of the second block (9) being removable.

3. System according to any of the preceding claims wherein the tanks (8) being placed at the level of the start of the escalator or moving walkway, or directly on the floor of the escalator or moving walkway, or on a base of a structure supporting the system, or on the support structure of the moving walkway or escalator.

4. System according to any of the preceding claims wherein the system presenting at least one upper side (2) and at least one lower side (3) for protection and/or advertising.

5. System according to any of the preceding claims wherein the fabrics (4) being resistant to friction, having the capacity to be impregnated, and being highly hydrophilic with sensing capability and incorporation and/or integration of antimicrobial nanoparticles.

6. System according to any the preceding claims wherein the fabrics (4) being made of taffeta, serge, or satin.

7. System according to any of the preceding claims, wherein the fabrics (4) comprising a fabric of the first block (4.1) and a fabric of the second block (4.2).

8. System according to any the preceding claim wherein the fabric of the first block (4.1) having a higher roughness than the fabric of the second block (4.2).

9. System according to any of claims 7 or 8 wherein the fabric of the first block (4.1) being non-abrasive to the surface of the handrail (1).

10. System according to any of the preceding claims wherein the shafts of the first block (5) and the shafts of the second block (9) being adjustable by means of screws and rails that are combined.

11. System according to any of the preceding claims wherein the light source (11) and the al least heat source (12) being powered by at least one electric motor (6).

12. System according to any of the preceding claims wherein the at least heat source (12) being at least one electrical resistance.

13. System according to any of the preceding claims wherein the system being horizontally adjustable through the distance between the constituents of the cleaning module (A).

14. System according to any of the preceding claims wherein the system further comprising angle posts and pre-drilled bars made of metal or any other type of material, joined and secured by bolted connections comprising screws, nuts, and washers.

15. System according to any of the preceding claims wherein the system being made of construction steel, plastic, or by composite materials produced from recycling undifferentiated plastic materials reinforced with plant fibres.

## Patentansprüche

1. Reinigung und Desinfektionssystem für Handläufe von Fahrtreppen und Fahrsteigen, wobei das System umfasst: ein Reinigungsmodul (A), ein Modul zur Behandlung der Oberfläche eines Handlaufs mit ultraviolettem Licht (B) sowie ein Trocknungsmodul (C), wobei:
a. das Reinigungsmodul (A) zwei Blöcke aufweist:
i. einen ersten Block, umfassend ein Paar Wellen des ersten Blocks (5), bestehend aus einer oberen Welle des ersten Blocks (5.1) und einer unteren Welle des ersten Blocks (5.2), und einen zweiten Block, umfassend ein Paar Wellen des zweiten Blocks (9), bestehend aus einer oberen Welle des zweiten Blocks (9.1) und einer unteren Welle des zweiten Blocks (9.2), wobei die Wellen die Rotation von jeweils einer Geweberolle (4) mittels Antriebsriemen (10) je Block bewirken, die die Wellen des ersten Blocks (5) mit den Wellen des zweiten Blocks (9) verbinden und durch mindestens einen Elektromotor (6) angetrieben werden, der synchron zur Bewegung der Fahrtreppe oder des Fahrsteigs arbeitet, um das Auf- bzw. Abwickeln der Gewebe (4) zu ermöglichen;
ii. Behälter (8), die mit Schläuchen (7) verbunden sind, wobei letztere Injektoren aufweisen, welche die Gewebe (4) besprühen;
iii. die Wellen des ersten Blocks (5) und die Wellen des zweiten Blocks (9) vertikal angeordnet sind, sodass die Gewebe (4) beim Bewegen mit der Oberfläche eines Handlaufs (1) in Kontakt kommen und zunächst grobe Verunreinigungen von der Oberfläche des Handlaufs (1) entfernen und anschließend die Oberfläche des Handlaufs (1) desinfizieren;
b. das Modul zur Behandlung der Handlaufoberfläche mit ultraviolettem Licht (B) umfasst mindestens eine UV-Lichtquelle (11), die so angeordnet ist, dass ihre Wirkung nach der durch das Reinigungsmodul (A) erfolgten Desinfektion und vor der Einwirkung des Trocknungsmoduls (C) eintritt, wobei das Trocknungsmodul (C) mindestens einen Trockner oder mindestens eine Wärmequelle (12) umfasst.

2. System nach dem vorhergehenden Anspruch, **dadurch gekennzeichnet, dass** die Wellen des ersten Blocks (5) und des zweiten Blocks (9) herausnehmbar sind.

3. System nach einem der vorhergehenden Ansprüche, **dadurch gekennzeichnet, dass** die Behälter (8) wahlweise am Anfang der Fahrtreppe oder des Fahrsteigs, direkt auf dem Boden der Fahrtreppe oder des Fahrsteigs, auf einer Basis einer das System tragenden Struktur oder auf der Trägerstruktur der Fahrtreppe bzw. des Fahrsteigs angeordnet sind.

4. System nach einem der vorhergehenden Ansprüche, **dadurch gekennzeichnet, dass** das System mindestens eine obere Abdeckung (2) und mindestens eine untere Abdeckung (3) zur Schutz- und/oder Werbezwecken aufweist.

5. System nach einem der vorhergehenden Ansprüche, **dadurch gekennzeichnet, dass** die Gewebe (4) reibungsbeständig sind, die Fähigkeit zur Imprägnierung besitzen, stark hydrophil sind und mit Sensorik sowie mit antimikrobiellen Nanopartikeln versehen bzw. integriert sind.

6. System nach einem der vorhergehenden Ansprüche, **dadurch gekennzeichnet, dass** die Gewebe (4) aus Taft, Serge oder Satin bestehen.

7. System nach einem der vorhergehenden Ansprüche, **dadurch gekennzeichnet, dass** die Gewebe (4) ein Gewebe des ersten Blocks (4.1) und ein Gewebe des zweiten Blocks (4.2) umfassen.

8. System nach einem der vorhergehenden Ansprüche, **dadurch gekennzeichnet, dass** das Gewebe des ersten Blocks (4.1) eine höhere Rauheit aufweist als das Gewebe des zweiten Blocks (4.2).

9. System nach einem der Ansprüche 7 oder 8, **dadurch gekennzeichnet, dass** das Gewebe des ersten Blocks (4.1) nicht abrasiv gegenüber der Oberfläche des Handlaufs (1) ist.

10. System nach einem der vorhergehenden Ansprüche, **dadurch gekennzeichnet, dass** die Wellen des ersten Blocks (5) und des zweiten Blocks (9) mittels Schrauben und Führungsschienen verstellbar sind.

11. System nach einem der vorhergehenden Ansprüche, **dadurch gekennzeichnet, dass** die Lichtquelle (11) sowie die mindestens eine Wärmequelle (12) durch mindestens einen Elektromotor (6) mit Energie versorgt werden.

12. System nach einem der vorhergehenden Ansprüche, **dadurch gekennzeichnet, dass** die mindestens eine Wärmequelle (12) mindestens eine elektrische Heizwendel ist.

13. System nach einem der vorhergehenden Ansprüche, **dadurch gekennzeichnet, dass** das System horizontal verstellbar ist durch Veränderung des Abstands zwischen den Komponenten des Reinigungsmoduls (A).

14. System nach einem der vorhergehenden Ansprüche, **dadurch gekennzeichnet, dass** das System zusätzlich Eckpfosten und vorgebohrte Stäbe aus Metall oder einem anderen geeigneten Material umfasst, die mittels Schraubverbindungen, bestehend aus Schrauben, Muttern und Unterlegscheiben, verbunden und befestigt sind.

15. System nach einem der vorhergehenden Ansprüche, **dadurch gekennzeichnet, dass** das System aus Baustahl, Kunststoff oder aus Verbundmaterialien besteht, die aus recycelten, nicht sortenrein getrennten Kunststoffen bestehen und mit Pflanzenfasern verstärkt sind.

## Revendications

1. Système de nettoyage et de désinfection pour mains courantes des escaliers mécaniques et des trottoirs roulants, dans lequel le système comprend un module de nettoyage (A), un module de traitement de la surface de la main courante au moyen de la lumière ultraviolette (B), et un module de séchage (C), dans lequel:
a. le module de nettoyage (A) comprend deux blocs:
i. un premier bloc, comprenant une paire d'axes du premier bloc (5), qui comprend un axe supérieur du premier bloc (5.1) et un axe inférieur du premier bloc (5.2), un second bloc comprenant une paire d'axes du second bloc (9), qui comprend un axe supérieur du second bloc (9.1) et un axe inférieur du second bloc (9.2), qui permet la rotation de deux bobines en (4) tissu, sur chaque bloc, au moyen de courroies d'entraînement (10) qui relient les axes du premier bloc (5) aux axes du second bloc (9) et qui sont entraînées par au moins un moteur électrique (6) synchronisé avec la rotation de l'escalier mécanique ou du trottoir roulant, assurant ainsi l'enroulement/le déroulement des tissus (4);
ii. des réservoirs (8) reliés à des tuyaux (7) qui disposent d'injecteurs couplés qui vaporisent les tissus (4);
iii. les axes du premier bloc (5) et les axes du second bloc (9) sont en position verticale de telle manière que les tissus (4), lors de leur déplacement, viennent au contact de la surface d'une main courante (1), respectivement retirant ainsi les gros débris de la surface de la main courante (1) et ensuite désinfectant la surface de la main courante (1) ;
b. le module de traitement de la surface de la main courante au moyen de la lumière ultraviolette (B), qui comprend au moins une source de lumière ultraviolette (UV) (11) et située de telle façon que son action se produit après l'étape de désinfection effectuée par le module de nettoyage (A) et avant l'action du module de séchage (C), ledit module de séchage (C) comprenant au moins un sécheur ou au moins une source de chaleur (12).

2. Système selon la revendication précédente, dans lequel les axes du premier bloc (5) et les axes du second bloc (9) sont amovibles.

3. Système selon l'une quelconque des revendications précédentes, dans lequel les réservoirs (8) sont placés au niveau du départ de l'escalier mécanique ou du trottoir roulant, ou directement sur le sol de l'escalier mécanique ou du trottoir roulant, ou sur une base d'une structure soutenant le système, ou sur la structure de soutien du trottoir roulant ou de l'escalier mécanique.

4. Système selon l'une quelconque des revendications précédentes, dans lequel le système présente au moins une partie supérieure (2) et au moins une partie inférieure (3) à des fins de protection et/ou de publicité.

5. Système selon l'une quelconque des revendications précédentes, dans lequel les tissus (4) sont résistants au frottement, ont la capacité d'être imprégnés, et sont très hydrophiles avec une capacité de détection et d'incorporation et/ou d'intégration de nanoparticules antimicrobiennes.

6. Système selon l'une quelconque des revendications précédentes, dans lequel les tissus (4) sont faits de taffetas, de serge ou de satin.

7. Système selon l'une quelconque des revendications précédentes, dans lequel les tissus (4) comprennent un tissu du premier bloc (4.1) et un tissu du second bloc (4.2).

8. Système selon l'une quelconque des revendications précédentes, dans lequel le tissu du premier bloc (4.1) présente une rugosité supérieure à celle du tissu du second bloc (4.2).

9. Système selon l'une quelconque des revendications 7 ou 8, dans lequel le tissu du premier bloc (4.1) est non abrasif pour la surface de la main courante (1).

10. Système selon l'une quelconque des revendications précédentes, dans lequel les axes du premier bloc (5) et les axes du second bloc (9) sont réglables au moyen de vis et de glissières qui sont combinées.

11. Système selon l'une quelconque des revendications précédentes, dans lequel la source de lumière (11) et au moins une source de chaleur (12) sont alimentées par au moins un moteur électrique (6).

12. Système selon l'une quelconque des revendications précédentes, dans lequel au moins une source de chaleur (12) est au moins une résistance électrique.

13. Système selon l'une quelconque des revendications précédentes, dans lequel le système est réglable horizontalement sur toute la distance entre les composants du module de nettoyage (A).

14. Système selon l'une quelconque des revendications précédentes, dans lequel le système comprend par ailleurs des poteaux d'angle et des barres pré-percées en métal ou tout autre type de matériau, reliés et fixés à l'aide d'assemblages boulonnés comprenant des vis, des écrous et des rondelles.

15. Système selon l'une quelconque des revendications précédentes, dans lequel le système est constitué d'acier de construction, de plastique ou de matériaux composites issus de matériaux plastiques indifférenciés de recyclage et renforcés par des fibres végétales.
